# EUROPEAN PATENT SPECIFICATION

(11) **EP 4 621 713 B1**
(45) Date of publication and mention of the grant of the patent: **11.11.2026**
(21) Application number: 25164359.9
(22) Date of filing: 18.03.2025
(51) Int. Cl.: G06T 7/12

(54) **ARTIFICIAL INTELLIGENCE-ASSISTED CONTOURING IN MEDICAL IMAGING**
DURCH KÜNSTLICHE INTELLIGENZ UNTERSTÜTZTE KONTURIERUNG IN DER MEDIZINISCHEN BILDGEBUNG
CONTOURNAGE ASSISTÉ PAR INTELLIGENCE ARTIFICIELLE EN IMAGERIE MÉDICALE

(30) Priority: 18.03.2024 US 202418607989
(43) Date of publication of application: 24.09.2025
(73) Proprietor: Siemens Medical Solutions USA, Inc., Malvern, PA 19355 (US)
(72) Inventor: ZHANG, Yue, Short Hills, NJ, 07078 (US)
(74) Representative: HKW Intellectual Property PartG mbB

(56) References cited:
- US-A1- 20240 041 430
- RAGAVIE PIRABAHARAN ET AL: "Interactive segmentation using U-Net with weight map and dynamic user interactions", ARXIV.ORG, CORNELL UNIVERSITY LIBRARY, 201 OLIN LIBRARY CORNELL UNIVERSITY ITHACA, NY 14853, 18 November 2021 (2021-11-18), XP091102609
- HE KELEI ET AL: "MetricUNet: Synergistic image- and voxel-level learning for precise prostate segmentation via online sampling", MEDICAL IMAGE ANALYSIS, OXFORD UNIVERSITY PRESS, OXOFRD, GB, vol. 71, 23 March 2021 (2021-03-23), XP086588712, ISSN: 1361-8415, [retrieved on 20210323], DOI: 10.1016/J.MEDIA.2021.102039
- MARINOV ZDRAVKO ET AL: "Guiding the Guidance: A Comparative Analysis of User Guidance Signals for Interactive Segmentation of Volumetric Images", 1 October 2023, MEDICAL IMAGE COMPUTING AND COMPUTER ASSISTED INTERVENTION - MICCAI 2023; [LECTURE NOTES IN COMPUTER SCIENCE; LECT.NOTES COMPUTER], SPRINGER NATURE SWITZERLAND, CHAM, PAGE(S) 637 - 647, ISBN: 978-3-031-43897-4, ISSN: 0302-9743, XP047671080

## Description

### BACKGROUND

The present embodiments relate to segmentation in medical imaging. Structure contouring is a fundamental task in medical imaging. This segmentation allows the user to observe a region of interest with focus and/or measure various attributes of the structure. The segmentation provides aid in diagnosis and treatment planning.

Clinicians commonly manually perform contouring of arbitrary selected structures. Structure contouring is task and user dependent. Taking contouring of left atrial appendage (LAA) in intracardiac echocardiography (ICE) as an example, the sonographer may delineate the contour tightly to the sharp image boundary of LAA in a diagnostic setting. In an interventional setting, the physician may tend to over contour the structure with more inclusive surrounding tissues to account for potential structure deformations over time (either a cardiac cycle or time from device making to implanting) for safety purposes. A given model or process for segmentation may not segment differently for the different purposes. The user may then manually alter the segmentation in a time consuming and user dependent fashion.

In the field of interactive segmentation, a user adjusts the contour. Artificial intelligence (AI) has shown promising performance on image segmentation. AI may assist in interactive segmentation. The user input is transformed into an image map, such as a Gaussian heatmap, Euclidean distance map, geodesic distance map, or simply a small constant disk. These maps are fed together with the initial image to generate a segmentation based on the user change. When users do more editing for modification, maps of the same kind are generated and used to generate updated segmentation. These approaches focus only on the correction, loosing information as to why the user made the correction.

US 2024/041430 A1 provides systems and methods for defining a boundary of a region of interest in an ultrasound image. The method includes receiving an ultrasound image having pixels and automatically processing the ultrasound image to output a probability of each of the pixels being in a region of interest. The method includes applying a first threshold value to determine a boundary of the region of interest. The first threshold value corresponds with a first segmentation sensitivity level of a plurality of segmentation sensitivity levels. The method includes displaying the ultrasound image with the boundary overlaid on the ultrasound image. The method includes receiving a user selection of a second segmentation sensitivity level that corresponds with a second threshold value different from the first threshold value, and dynamically updating the boundary overlaid on the ultrasound image at the display based on the second threshold value.

Ragavie Pirabaharan and Naimul Khan: "Interactive segmentation using U-Net with weight map and dynamic user interactions", arXiv: 2111.09740, 18. November, 2021, propose a novel interactive segmentation framework, where user clicks are dynamically adapted in size based on the current segmentation mask. The clicked regions form a weight map and are fed to a deep neural network as a novel weighted loss function. To evaluate a loss function, an interactive U-Net (IU-Net) model which applies both foreground and background user clicks as the main method of interaction is employed. The authors train and validate on the BCV dataset, while testing on spleen and colon cancer CT images from the MSD dataset to improve the overall segmentation accuracy in comparison to the standard U-Net using their weighted loss function.

### SUMMARY

The invention is defined by the attached set of claims. Further details of the disclosed method, devices and system are described below, which are helpful for understanding the claimed invention.

Systems, methods, and non-transitory computer readable media are provided for AI-assisted segmentation. When a user alters a segmentation of an object, indications of what is not desired (e.g., samples from where the segmentation border use to be but is no longer due to the change) are used to inform segmentation for non-changed regions. Negative and positive samples from a user alteration may be extracted. Information from these negative, with or without the positive samples, is used by a machine-learned model to re-segment the object. The user change is used to correct the segmentation even for non-changed regions, minimizing the amount of manual adjustment needed and providing segmentation specific to the user and/or purpose.

In a first aspect, a method is provided for segmentation assistance in a medical imaging system. A medical image, such as an ultrasound image, of a patient is acquired. An object in the medical image is segmented, resulting in a first contour for the object. A change to a part of the first contour for the object is received from a user input. Positive and negative samples are extracted from the medical image based on the changed contour. The object in the medical image is segmented by input of the ultrasound image and information for the positive and negative samples to a first machine-learned model. The segmentation results in a second contour. An image of the second contour is generated.

In a second aspect, a medical system is provided for interactive segmentation. A memory is configured to store a medical image. A user input is configured to receive an alteration from a user. An image processor is configured to receive an alteration from a first location to a second location for a first segmentation of the medical image from the user input and use first patches about the first location and second patches about the second location for a second segmentation of the medical image. The second segmentation is formed by a machine-learned network. A display is configured to display a segmentation image of the second segmentation. The image processor may implement the first aspect.

In a third aspect not covered by the attached claims, a method is provided for AI-assisted contouring in a medical imaging system. A map is generated. The map represents (a) similarity of locations in a medical image to a first position of a first contour of an object in the medical image after a change to the first contour and (b) dissimilarity of the locations to a second position of the first contour of the object in the medical image before the change. The object is segmented by a machine-learned model in response to input of the medical image and the map to the machine-learned model. The segmented object is displayed.

The illustrative embodiments listed below summarize other features or aspects. Any one or more of the aspects described above or in the illustrative embodiments may be used alone or in combination with other of the illustrative embodiments, features, or aspects, e.g., as depicted in the claims. Any aspects or features of one of method, system, or computer readable media may be used in the others of method, system, or computer readable media. These and other aspects, features and advantages will become apparent from the following detailed description of preferred embodiments, which is to be read in connection with the accompanying drawings. The present invention is defined by the following claims, and nothing in this section should be taken as a limitation on those claims. Further aspects and advantages of the invention are discussed below in conjunction with the preferred embodiments and may be later claimed independently or in combination.

### BRIEF DESCRIPTION OF THE DRAWINGS

The components and the figures are not necessarily to scale, emphasis instead being placed upon illustrating the principles of the embodiments. Moreover, in the figures, like reference numerals designate corresponding parts throughout the different views.
Figure 1 is a flow chart diagram of one embodiment of a method for AI-assisted contouring in medical imaging;
Figure 2 is a flow chart diagram of another embodiment of the method for AI-assisted contouring;
Figure 3 is a flow chart diagram of one embodiment of use of positive and negative samples from a user change for segmentation by AI;
Figure 4 illustrates a user change to a contour;
Figure 5 illustrates a progression of segmentations based on manual and AI-assisted alterations; and
Figure 6 is a block diagram of one embodiment of a medical system for AI-assisted interactive contouring.

### DETAILED DESCRIPTION OF EMBODIMENTS

Given an initial contour (e.g., produced by AI), the user only needs to adjust the contour locally. Negative with or without positive samples of the adjustment reflect the user's intention for the remainder of the contour. The samples are used by the AI or another AI to understand the user's intention and adjust the entire contour globally.

In an example used herein, interactive contouring is provided for anatomical structures in three-dimensional (3D) or four-dimensional (4D) ultrasound. Starting with a single user click, the system first aims to produce an initial contour of the target with a spatial-temporal AI segmentation model. If the user disagrees, the user refines a local segment of this initial contour. A refinement model then generates positive and negative samples (image patches around the corrected segment) from the initial contour and the manually corrected segment of contour. A matching approach for the positive and negative samples is used by a deep network model to efficiently screen and update the rest of the contour (e.g., segmentation is performed using the image and information from the samples of the change).

Rather than only focusing on encoding the segment as corrected (i.e., positive sample), the negative or both the negative and positive samples are used to learn about the user's intention and update the contour globally to be consistent with this intention. The user edit (positive samples) and also the reason of the editing by looking at the negative samples are used. With a contrastive weight map from the positive and negative samples, the proposed approach generates high attention (e.g., high weight score in the contrastive weight map) on the patches (positive) that are of high similarities with the user intent to contour and low attention on the patches (negative) that user modifies on.

Rather than feeding the edited contour and original image through a segmentation network, a relational map encoding may be used. The relational map encoding is based on the positive and negative samples, creating a challenge for the machine learning network to learn the intension of the user. The network does not know the part of the image on which it should focus. By machine training with the contrastive weight map, the image patches that the network should put attention on and image patches that the network should not focus on give the network explicit instruction in intention. The resulting machine-learned model is trained to use the positive and negative samples for any localized change in segmentation to inform global segmentation of the object.

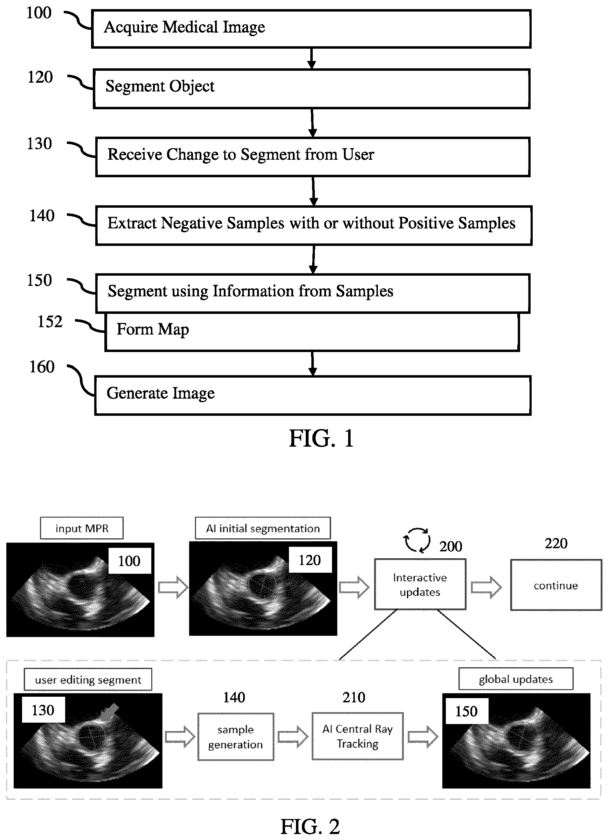

Figure 1 is a flow chart diagram of one embodiment of a method for segmentation assistance in a medical imaging system. The method uses AI-assisted contouring. Negative samples, with or without positive samples, of a change to a segmentation are extracted to reflect the user's intention for where the contour should not be with or with where the contour should be. AI uses the samples and the medical image to segment again based on the user's intention.

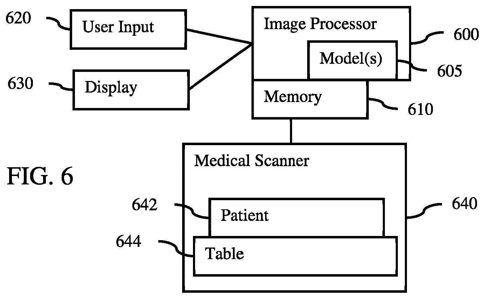

The method of Figure 1 is performed by a medical system, such as the medical scanner or image processor of Figure 6 or another medical system. For example, an ultrasound scanner acquires an image. The image processor segments, receives input from a user input for a change, extracts samples from the change, and generates an image of an updated segmentation. The image processor may generate an image. A display, based on an image created by the image processor, displays the image.

The method is performed in the order shown (e.g., top to bottom or numerical), but other orders may be used. Additional, different, or fewer acts may be provided. For example, act 120 is not provided where the acquired image includes the segmentation, such as previously performed. As another example, act 152 is not used, such as where the samples are directly input without mapping. In another example, act 160 is not performed, such as where the refined segmentation is stored in a patient medical record for later viewing. In yet another example, acts for configuring the scanning and/or use of output segmentation information are performed.

Figure 2 is a flow chart diagram of one implementation of the method of Figure 1 in one context or workflow. Figures 1 and 2 will be described together. Other contexts or workflows for the method of Figure 1 may be used. In Figure 2, the workflow includes interactive contouring for ultrasound images. Starting with a two-dimensional (2D) image, such as a 2D multi-planar reconstruction (MPR) from a 4D ultrasound sequence, an AI module produces an initial contour of the target in act 120. Then, the editing loop starts at the interactive updates of act 200. The user edits a segment of the contour in act 130 to include more or less neighboring soft tissue. A sample generation module generates positive and negative samples (e.g., image patches) around the editing area in act 140. An AI module then takes these samples as input, producing an updated contour result globally in act 150 based on ray tracking sampling of the image space in act 210. If the user performs more editing, this loop continues in act 220 until satisfaction.

In act 100, a medical scanner (e.g., ultrasound scanner or imaging system) or image processor acquires a medical image representing a patient. The patient is scanned. The resulting image is the medical image. In other embodiments, the medical image for a patient is acquired from memory or transfer over a computer network.

The medical image represents any part of the patient, such as representing an organ, head, or torso of the patient. In one implementation, the medical image is an ICE image acquired with an ICE transducer (e.g., transducer on an ICE catheter scanning from within the heart of the patient). This ultrasound image represents a heart region of the patient, such as imaging an ostium, heart chamber, and/or valve of the patient, and/or device within the patient. ICE delivers real-time, high-resolution visualization of cardiac structures and provides continuous monitoring of catheter positioning within the heart. Procedural complications, such as pericardial effusion or thrombus formation, may be detected early using ICE. ICE exhibits exceptional patient tolerance, reduces fluoroscopy duration, and eliminates the necessity for general anesthesia or a second operator. Other ultrasound imaging may be used, such as transesophageal echocardiography (TEE). In other implementations, the medical image is a magnetic resonance image, an ultrasound image, a computed tomography image, or an x-ray image.

The medical image and corresponding segmentation have any spatial extent, such as 2D or 3D. Temporal variation may be provided, such as segmenting through a sequence of images over one or more heart cycles. 2D examples will be used herein. A static 2D image example is used herein.

The medical image is data arranged as pixels or voxels and may or may not be formatted for display. The medical image may be data acquired by scanning with or without further processing (e.g., scan conversion). The medical image may be data used to generate a displayed image representing the patient or may be a displayed image.

In act 120, the image processor segments an object in the ultrasound image. The segmentation identifies a border, area, and/or volume corresponding to the object. The segmented object as a border, area, and/or volume has a contour or outer edge (curve or surface). The pixels, voxels, or scan locations corresponding to the object are identified as the segmentation. The segmentation results in a contour representing the outer edge of the object or boundary of the object with other objects.

Any object or objects may be segmented. For an ICE example, an ostium, valve, or chamber is segmented. Soft tissue structures (e.g., organs or muscles), bone, instruments, or implants may be objects represented in the medical image and segmented.

The segmentation is performed using any process or function, such as intensity thresholding with low pass filtering. In one implementation, full width, half maximum (FWHM) or intensity thresholding with various standard deviations is used to segment. Alternatively, a user (e.g., radiologist) or image processor segments. In another approach, a machine-learned model, such as an encoder-decoder-based neural network, outputs the segmentation or segmentations in response to input of the medical image. An image-to-image, U-Net, or encoder-decoder network trained to output the segmentation in response to input of medical image data may be applied. The machine-trained model (segmentor) generates values for features in hidden layers in response to input of the medical image and uses the values of the features to output the segmentation.

The segmentation outputs the identification of the locations occupied by the object. A contour (e.g., boundary, area distribution, or volume distribution) of the object is output as the segmentation. The output contour is based on or generated in response to the input or displayed medical image.

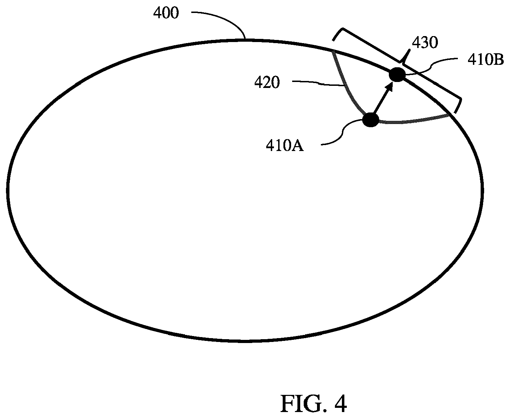

The segmentation is displayed to the user. Given user preferences and/or purpose for the segmentation, the user may alter the segmentation. For example, the user visually identifies one or more locations where the contour covers too much or not enough of the object. Figure 4 shows an example. The initial segmentation 400 is generally oval but includes a notch or indentation formed by the contour 420 in the part 430. As another example, a segmentation of the LAA is not tight to the boundary. Since the physician is taking measurements or making a diagnosis, the contour should be tight to the LAA boundary. In yet another example, the segmentation of the LAA is tight to the boundary. Since the physician is planning or performing an interventional procedure, the contour is desired to be more inclusive to account for potential structure deformations.

In act 130, the image processor receives a change to the segmentation. The contour is altered. For example, just part of the contour is changed.

The alteration may be automated, such as using another machine-learned model to identify a change. In one approach for interaction with the user, the alteration is received from the user through a user input. The user uses click-and-drag or other user interface operations with a user input (e.g., mouse, trackball, touch pad, touch screen, and/or keyboard) to change the contour. In the example of Figure 4, the user selects a point or location 410A along the contour 420. The user then drags the point or location 410A to the desired point or location 410B. For just a part 430 of the contour 420, the contour 420 is altered to segment based on this change. The contour may have a defined elasticity, using a physics model, or by another function relating the change in a point or region to adjacent parts (i.e., within the part 430) of the contour 420. Other parts of the contour 420 do not change, such as outside the part 430.

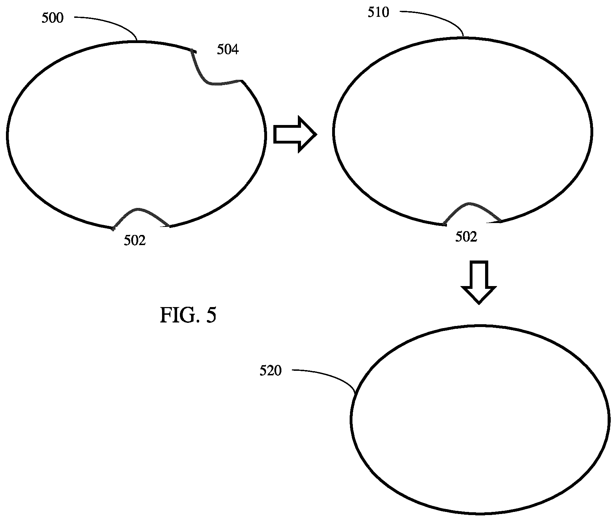

The user selects the location 410A as a representative change. Rather than changing all the locations desired, one or more representative changes are made. For this representative region, one or more locations of points are shifted, such as inward or outward relative to the object. Other parts of the contour are unchanged (free of change) by user input. Figure 5 shows an example. The initial contour 500 from the segmentation of act 120 includes two indentations 502, 504. The user selects a point in the indentation 504 to change the contour. After the change, the contour 510 results where the notch 504 no longer exists but the notch 502 still exists. While shown as notches, any combination of notches and/or protrusions may occur.

The change or alternation indicates the intention of the user for the current segmentation. In act 140, the image processor extracts positive and/or negative samples from the image based on the changed contour. The image processor uses information from the medical image based on the change to indicate the intention of the user for the segmentation.

Samples are extracted from the medical image, such as the ultrasound image. The samples are patches of any size, such as 4x8, 8x8, or other sized or shaped patches. Other samples, such as a sparse sampling, may be obtained. The samples may be directional, such as having a longer extent parallel with or perpendicular to the contour.

Any number of samples may be extracted. For example, one or more positive samples and one or more negative samples are extracted. The resolution of the medical image and/or size of the part 430 may control or influence the number of samples. For example, a sample is extracted for every point along the contour of the change part 430 at the image resolution. A set number of samples may be obtained, such as sampling along the contour after dividing the changed part into the set number.

Positive samples may be extracted. For the part 430 of the contour 400 that is changed, samples along the contour after the change are extracted. The user altered the segmentation so that the contour is along the desired tissue. Samples extracted from the contour 420 after the change reflect positive placement of the contour 400.

Negative samples are extracted. For the part 430 of the contour 400 that is changed, samples along the contour 420 before the changes are extracted. The user altered the segmentation so that the contour 400 is not along undesired tissue. Samples extracted from the contour 400 for the part 430 before the change reflect negative or undesired placement of the contour 400. Other undesired or negative samples may be extracted, such as at tissue centered away from the contour (e.g., inward or outward placement from the contour relative to the object but not along the contour after change).

In one implementation, there are two segments of the contour: segment that has been edited and the remaining part. For the edited segment, image patches centered around the contour as changed are extracted. The user intends to move contour to these kind of patches, thus they are positive samples. Image patches centered are extracted around the original corresponding contour segment before the edit. The user intends to not have the contour over these patches, thus they are negative samples.

The extracted samples represent samples showing the tissue locations for the contour and not for the contour. These samples may be used to assist in identifying contour locations in other parts of the contour. The ultrasound image may be sampled in any manner to test for segment location. In one approach shown in Figure 2, a radial sampling is used. The center of the object as initially segmented is used as a center for radial lines. The contour location along each radius line is found by sampling along the radius line. The extracted samples may be used to assist in the identification of the location along the radius line corresponding to the radii. For the remaining segment that the user does not edit, radial patches centered around the contour may be extracted. The positive and negative samples are used to identify, for each ray or radius line, which patch along the ray should be a suitable patch on which to lay the contour. Other sampling of the medical image may be, such along a Cartesian grid.

In act 150, the image processor segments the object in the medical (e.g., ultrasound) image. The segmentation results in another contour for the same object, where the contour is likely but not necessarily different from the previous contour from the segmentation of act 120. This subsequent segmentation of act 150 uses the medical image and information from the samples as inputs to create the contour. The contour is provided as a border, area, volume, or other designation of locations for the object distinguished from other locations.

The segmentation is for the parts not changed by the user. The entire contour or object may be segmented, providing the contour for the part changed and other parts. The information from the samples over the medical image is used to identify the contour (segmentation) for all or other parts of the object despite not having any user input specifically for those other parts. Figure 5 shows an example where the initial contour 500 is altered by the user, resulting in contour 510. In act 150, the image processor uses the samples from the user change and the medical image to segment the object again, resulting in the contour 520. The contour 520 does not include the indentation 502 and does not include the indentation 504.

The same object is to be segmented again after the change of act 130. The samples from the change are used for this re-segmentation. The samples are either input directly as information to the segmentation process or used to derive further information, which further information is input directly to the segmentation process. Act 152 shows one way (generating a map) in which further information is derived for input to the segmentation process.

Any of the segmentation processes or approaches discussed above for act 120 may be used in the segmentation of act 150. The same or different approach is used in act 150 than for act 120. In one implementation, the segmentation of act 150 uses a machine-learned model with inputs for the medical image and information from the samples. The segmentation is generated by the machine-learned model in response to input on two or more input channels, one for the medical image and another for information from the samples related to the change made by the user.

The machine-learned model was previously trained using training data. The model is formed from an architecture defining learnable parameters. The training data included many samples of inputs and corresponding ground truth (e.g., correct outputs), such as expert annotated segmentations formed from a database of patient scans.

For training the machine learning model, the machine learning model arrangement (architecture) is defined. Any now known or later developed machine-learned model may be used. For example, an image-to-image network, U-Net, DenseNet, ResNet, or encoder-decoder network is used. Down sampling layers, convolutional layers, pooling layers, dropout layers, skip connections, up sampling layers, and/or other neural network layers may be used. Any architecture that receives image (spatial) information to output spatial (e.g., image) information may be used. The definition is by configuration or programming of the learning. The number of layers or units, type of learning, and other characteristics of the model are controlled by the programmer or user. In other embodiments, one or more aspects (e.g., number of nodes, number of layers or units, or type of learning) are defined and selected by the machine during the learning. Training data, including many samples of the input data and the corresponding output, is used to train. The relationship of the input to the output is machine learned.

The image processor or another processor machine trains the model. The training learns values for learnable parameters (e.g., weights, connections, filter kernels, and/or other learnable parameters of the defined architecture). Deep or another machine learning may be used. The weights, connections, filter kernels, and/or other parameters are the features being learned. For example, convolution kernels are features being trained. Using the training data, the values of the learnable parameters of the model are adjusted and tested to determine the values leading to an optimum estimation of the output given an input. Adam or another optimization is used to train.

During training, the loss is minimized. The loss is derived from comparison of the output of the model to an expected output (i.e., ground truth). The loss function is a L1, L2, or other error function between the network output and the ground truth of the training data. Other losses may be used. Using optimization, different values of the learnable parameters are tested across different samples of the training data to minimize the loss (or maximize a reward).

In one implementation, the machine-learned model includes an input for information from the samples, including the negative samples or samples from where the contour was before change by the user and is not after the change by the user. The input is the samples themselves or information derived from them. For example, a map is derived from the samples in act 152. The map indicates user intention for the location of the change and/or other locations. For example, the map compares the extracted samples to the medical image at locations sampled throughout the medical image. The sampling may be limited to within a threshold distance (e.g., 10% or 10 mm) of the initial contour from act 120 or the entire image may be fully or sparsely sampled. The results of the comparison, such as similarity, are used to derive a contrast value or weight for each location. In the radial lines sampling implementation, various radial patches are sampled along each of various radial lines. Each position along each of the radial lines is sampled. The sampling may be limited to being within a threshold distance from the previous contour. A patch centered on each sample location is extracted or defined.

The radial or sample patches are compared to the negative samples. Greater similarity or lesser dissimilarity indicates that the contour should not be located there. Comparison with the positive samples may also be used. Greater similarity or lesser dissimilarity of a radial patch with the positive samples indicates that the contour should be located there. Where multiple positive or negative samples are used, an average measure may be used. Where both positive and negative samples are used, a combination of results of the comparison may be used. Any combination may be used, such as a weighted difference or a ratio. The map is generated in act 152 as weights or relational values by sample location of the medical image to the positive and/or negative samples from the change locations. Different locations along or around the object are sampled and compared, generating a map of a spatial distribution of similarity (dissimilarity) to the extracted negative and/or positive samples. The map may then be used as an input to the machine-learned model.

In one approach for act 152, the image processor generates the map as representing (a) similarity of locations in a medical image to a position of a contour of an object in the medical image after a change to the contour (positive sample) and (b) dissimilarity of the locations to a position of the contour of the object in the medical image before the change (negative sample). The change to the contour by a user for a part of the contour is used to map for other parts of the contour that are unchanged by the user. The relational map is formed with the positive and/or negative samples. Where both positive and negative samples are used, a contrastive weight map is based on similarity of locations in the image with the positive samples and dissimilarity with the negative samples. Any combination of similarity and dissimilarity measures may be used. Any measure of similarity or dissimilarity may be used, such as minimum sum of absolute differences or cross correlation coefficient. The contrastive weight map is formed from the positive and negative samples, and the machine-learned model outputs a contour in response to input of the image and the contrastive weight map.

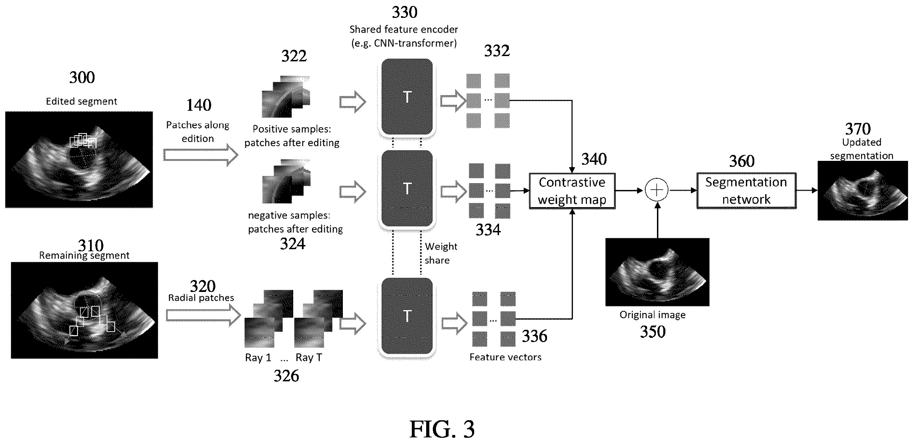

Figure 3 shows an example of map generation, in act 152, using the samples. Embedded features 332, 334, 336 of the samples are used in the comparison to form the contrastive weight map 340. The edited segment or contour 300 is used to extract, in act 140, the positive samples 322 as patches around the part of contour after editing. The part of the contour before editing is used to extract, in act 140, the negative samples 324 as patches around the contour prior to editing. Patches 326 are extracted in act 320 from the remaining parts of the segmentation, such as within a threshold distance from the previous segmentation along radial lines, and/or from a sampling of the medical image including at locations away from the part that is changed by the user.

The map uses embedded features 332, 334, 336 of the patches 322, 324, 326. Embedded features 332 derived from the positive samples (patches 322), embedded features 334 derived from the negative samples (patches 324), and embedded features 336 derived from the sample patches 326 are used to form the map 340. The embedded features 332, 334, 336 are formed or output by an encoder 330 or another neural network. The image processor generates a latent representation from the input patches 322, 324, 326. The patches 322, 324, 326 are input to the encoder 330. In response to input, the encoder 330 generates the latent representations 332, 334, 336 as a fingerprint.

The encoder 330 is a convolutional neural network and a transformer. In other approaches, other encoders formed by other neural networks may be used, such as a fully connected neural network or a convolutional neural network with a soft-max output layer or multi-layer perceptron. A deep learned neural network may be used for the encoder 330.

The encoder 330 operates based on how the encoder 330 was trained. Different training results in different values of learnable parameters, so results in different operation. In one approach, the encoder 330 or another machine-learned model for generating the latent representations for the patches 322, 324, 326 was trained with a decoder (e.g., the encoder 330 is trained as part of a segmentation network). Alternatively, the encoder 330 was trained with many samples of patches as training data where the ground truth is binary (0 or 1) for positive or negative. The encoder 330 is trained to distinguish between positive and negative input samples.

Once trained, the features 332, 334, 336 are extracted from layers of the encoder 330 prior to the output layer (e.g., prior to a softmax or multi-layer perceptron or prior to a decoder). For example, features from one or more layers after the input layer and before the output (i.e., binary 1 or 0) layer of the encoder 330 are used. Alternatively, the encoder 330 outputs a latent representation, such as where trained as part of an encoder-decoder. A latent representation formed by the encoder 330 is used as the feature vectors 332, 334, 336. Features from one or more hidden layers of the encoder 330 are used. The same encoder 330 with the same learned parameter values is used for each patch of each type. In alternative approaches, different encoders 330 are trained for different types (e.g., positive, negative, and image samples) of patches.

Given the positive and negative samples (patches 322 and 324) and sample (e.g., radial) patches 326 from the initial or previous segmentation, these patches 322, 324, 326 feed into the feature encoder 330 with shared weights for the different types of patches 322, 324, 326. Deep feature embeddings (feature vectors 332, 334, 446) are obtained for each patch.

The contrastive weight map 340 is built, in act 152, from these features. For each sample location with a corresponding patch 326, a score is formed from the features for that patch 326, features for the positive patches 322, and features for the negative patches 324. The score encourages similarity between the sample patch 326 and the positive sample patches 322 while discouraging similarity with the negative sample patches 324 but the similarity is based on embedded features. This map 340 then helps the downstream machine-learned model 360 (network) to put more attention on the highly scored regions and less on the lowly scored regions (or vise versa depending on how the map is generated). The weight map 340 is concatenated with the original image 350, which together are input to the segmentation network (machine-learned model 360 for segmentation), which outputs the updated segmentation 370.

In one implementation, the contrastive weight is a score that maximizes the similarity of the sample (e.g., radial) patch embedding 336 with the positive patch embedding 332 while minimizing the sample (e.g., radial) patch embedding 336 with the negative patch embedding 334. Given the positive samples *p*₁*,p*₂*, ..., pₘ,* negative samples *n*₁*,n*₂*, ..., nₛ,* and radial (sample) patches as *r*₁*,r*₂*, ... , r_{T}*, these image patches are input to the feature encoder 330, such as a convolutional neural network followed by a transformer encoder. ,f with a superscript denotes their feature embeddings 332, 334, 336, ${f}_{r}^{i} , {fp}^{j} , {f}_{n}^{k}$, accordingly. The contrastive weight for the *i* th radial patch 326 is defined as: ${W}_{i} = - log \frac{{max}_{j ∈ \left\{1,2,..,m\right\}} \left(exp\left(sim\left({f}_{r}^{i ,}{f}_{p}^{j}\right)/τ\right)\right)}{{\sum }_{k = 1}^{s} exp \left(\frac{sim \left({f}_{r}^{i}, {f}_{n}^{k}\right)}{τ}\right)} ,$ where *τ* is a temperature parameter. The numerator indicates the similarity of the radial patch (after encoding) with the positive examples, while the denominator indicates the dis-similarities of the radial patch with the negative samples. The weight factor or score for each radial patch 326 is determined. Other functions may be used. The weights for the contrastive weight map 340 are a spatial distribution for the sample patches 326 that instructs or informs the downstream segmentation network 360 to pay proper attention to the relevant part of the image. The weights distinguish possible positive from possible negative locations for the contour based on the change made by the user. The weight map is formed by calculating the score for all the sample (e.g., radial) patches 326. For each of different location samples from the image, a weight is formed from a maximization of similarity of the location sample with the positive samples as inversely weighted by similarity of the location sample with the negative samples.

The contrastive weight map 340 and image 350 are input to the machine-learned model (segmentation network 360), which outputs the contour in response. The segmentation is globally updated based on the local change made by the user. The machine-learned model 360 was trained with many samples of input images and information from extracted patches with ground truth segmentation. As shown in Figure 2, if the user desires more editing of the contour, this process is repeated until the final segmentation target of the user is reached.

In act 160 of Figure 1, the image processor generates an image. The image shows the segmentation (contour). The segmentation may be a graphic, such as a border line or shape of the object. The segmentation may be an alteration of the medical image, such as coloring or highlighting the object as segmented. In one approach, a graphic of the segmentation is overlaid on the medical image. In other approaches, a representation of the object is extracted from the image based on the segmentation. The representation of the object alone is displayed as the segmentation.

The segmented object is displayed to the user. The user may use the segmentation for measurement, diagnosis, therapy planning, or other purpose.

Figure 6 shows one embodiment of a medical system for interactive segmentation. The medical system includes the display 630, user input 620, memory 610, and image processor 600. The display 630, image processor 600, user input 620, and/or memory 610 may be part of the medical scanner 640 (e.g., ultrasound scanner), a computer, server, workstation, or other system for image processing medical images from a scan of a patient. A workstation or computer without the medical scanner 640 may be used as the medical system.

Additional, different, or fewer components may be provided. For example, a computer network is included for remote image generation of locally captured image data or for local prediction from remotely captured image data. The machine-learned models 605 are applied as standalone applications on the workstation or a local device or as a service deployed on network (cloud) architecture. In another example, the medical scanner 640 is not provided.

The image (e.g., ultrasound image), segmentation, user edits, values of learned parameters, machine-learned models 605, feature values (e.g., latent representation), positive samples, negative samples, image samples, display image, and/or other information are stored in a non-transitory computer readable memory, such as the memory 610. The medical image (e.g., magnetic resonance image, ultrasound image, computed tomography image, or x-ray image) is stored in the memory 610. The memory 610 is an external storage device, RAM, ROM, database, and/or a local memory (e.g., solid state drive or hard drive). The same or different non-transitory computer readable media may be used for the instructions and other data. The memory 610 may be implemented using a database management system (DBMS) and residing on a memory, such as a hard disk, RAM, or removable media. Alternatively, the memory 610 is internal to the processor 600 (e.g., cache).

The instructions for implementing the training or application processes, the methods, and/or the techniques discussed herein are provided on non-transitory computer-readable storage media or memories, such as a cache, buffer, RAM, removable media, hard drive, or other computer readable storage media (e.g., the memory 610). Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code, and the like, operating alone or in combination.

In one embodiment, the instructions are stored on a removable media device for reading by local or remote systems. In other embodiments, the instructions are stored in a remote location for transfer through a computer network. In yet other embodiments, the instructions are stored within a given computer, CPU, GPU, or system. Because some of the constituent system components and method steps depicted in the accompanying figures may be implemented in software, the actual connections between the system components (or the process steps) may differ depending upon the manner in which the present embodiments are programmed.

The user input 620 includes a keyboard, buttons, sliders, dials, trackball, mouse, touch pad, touch screen, and/or another device for receiving user input in a user interface. The user input is configured by the image processor 600 or another processor to receive an alteration from the user. A segmentation is displayed. The user interface is configured to allow the user to alter the segmentation, such as changing part of the contour. A position of a border of the segmentation is changed by the user based on received user input, such as from clicking and dragging interaction with the displayed contour. More than one change may be made.

The image processor 600 is a controller, control processor, general processor, micro-processor, tensor processor, digital signal processor, three-dimensional data processor, graphics processing unit, application specific integrated circuit, field programmable gate array, artificial intelligence processor, digital circuit, analog circuit, combinations thereof, or other now known or later developed device for processing image data. The image processor 600 is a single device, a plurality of devices, or a network of devices. For more than one device, parallel or sequential division of processing may be used. Different devices making up the image processor 600 may perform different functions. In one embodiment, the image processor 600 is a control processor or other processor of the medical scanner 640. The image processor 600 operates pursuant to and is configured by stored instructions, hardware, and/or firmware to perform various acts described herein.

The image processor 600 or another remote processor is configured to train machine learning architectures. Based on a user provided or other source of the network architecture and training data (e.g., formed from a database of patient scans and expert curation of ground truth), the image processor 600 learns to relate one or more input variables (e.g., image or image and information from negative samples) to outputs (e.g., segmentation or distinguishing positive from negative samples). The result of the training is a machine-learned model(s) 605, such as a model for generating a segmentation in response to input of negative samples or information derived therefrom and a medical image. Others of the machine-learned models discussed herein (e.g., encoder 330 or segmentation network for the initial segmentation without using information from samples for a user change) may be trained by the image processor 600 and/or stored in the memory 610.

Alternatively, or additionally, the image processor 600 is configured to apply the machine-learned model(s) 605 (i.e., apply a segmentation model with input of the medical image, apply an encoder to generate embedded features for patches, and/or apply a segmentation model with input of the medical information and information derived from positive and/or negative samples from a user change). The machine-learned models 605 may include a machine-learned network for global segmentation based on local user change.

The image processor 600 is configured to receive an alteration from one location to another location for a segmentation of the medical image. The alteration is received from the user input 620. The image processor 600 is configured to use patches about the one location and patches about the other location for another segmentation of the medical image. The other segmentation is formed by the machine-learned network or model 605. The patches about the one location are at locations of the border prior to the change by the user, and the patches about the other location are at locations of the border after the change by the user.

In one approach, the machine-learned network or model 605 is an image-to-image network (e.g., U-Net or encoder-decoder) with one input for the medical image and another input for a relational map relating positions in the medical image with similarity with the patches (negative) by the one location and the patches (positive) by the other location.

In a further approach, the image processor is configured to generate feature embeddings for the patches (patches about the one location, patches about the other location, and patches sampled from different positions in the medical image). The relational map is formed of weights maximizing similarity of the feature embeddings of patches at the different sample positions with the feature embeddings of the patches from the changed contour weighted by (e.g., divided by) similarity of the feature embeddings of the patches at the different sample positions with the feature embeddings of the patches from the contour prior to change by the user.

The image processor 600 is configured to generate an output, such as an image showing the segmentation. The contour of the object is displayed alone or with other information, such as an overlay on the medical image or an image of the object extracted from the medical image based on the segmentation. The image processor 600 is configured to generate a visualization of the segmentation (contour).

The display 630 is a CRT, LCD, projector, plasma, printer, tablet, smart phone or other now known or later developed display device for displaying the output, such as an image of the segmentation (segmentation image). Images for the previous segmentation, alteration, and/or segmentation after alteration by the user may be displayed in sequence and/or together.

The medical scanner 640 is a diagnostic or therapeutic scanner (e.g., ultrasound, computed tomography, x-ray, or magnetic resonance scanner). The scanner 640 operates pursuant to one or more settings to scan a patient 642 resting on a bed or table 644. The settings control scanning including transmission, reception, reconstruction, and image processing. A scanning protocol is followed to generate data representing the patient 642, such as an ICE ultrasound image representing a cardiac region of the patient. The patient 642 is imaged by the scanner 640 using the settings. The scanner 640 generates the medical image representing the object to be segmented.

Various improvements described herein may be used together or separately. Although illustrative embodiments of the present invention have been described herein with reference to the accompanying drawings, it is to be understood that the invention is not limited to those precise embodiments, and that various other changes and modifications may be affected therein by one skilled in the art without departing from the scope of the invention as defined by the claims.

## Claims

1. A method for segmentation assistance in a medical imaging system, the method comprising:
acquiring (100) a medical image (350) of a patient;
segmenting (120) an object in the medical image (350), the segmenting resulting in a first contour (420, 500) for the object;
receiving (130), from a user input, a change to a part (430) of the first contour (420, 500) for the object;
extracting (140) positive and negative samples (322, 324) from the medical image (350) based on the changed contour (400, 510);
segmenting (150) the object in the medical image (350), the segmenting resulting in a second contour (520) by input of the medical image (350) and information for the positive and negative samples (322, 324) to a first machine-learned model (360); and
generating (160) an image of the second contour (520).

2. The method of claim 1, wherein the medical image (350) comprises a magnetic resonance image, an ultrasound image, a computed tomography image, or an x-ray image.

3. The method of claim 2, wherein acquiring (100) a medical image (350) comprises acquiring (100) an intracardiac echocardiography, ICE, image with an ICE transducer, the ICE image (350) representing a heart region of the patient, wherein the object comprises an ostium, heart chamber, or valve.

4. The method of any one of claims 1 - 3, wherein segmenting (120) resulting in the first contour (500) comprises segmenting (120) by a second machine-learned model (605) outputting the first contour (500) in response to input of the medical image (350).

5. The method of any one of claims 1 - 4, wherein receiving (130) comprises receiving (130) a user input change shifting a location (410A) of a point on the first contour (420, 500) away from the first contour (420, 500), the part (430) comprising the location (410A) and adjacent locations shifted with the change where other parts of the first contour (400, 500) are free of the change.

6. The method of claim 5, wherein segmenting (150) comprises segmenting the entire first contour (420, 500), providing the second contour (400, 520) for the part (430) changed by the user and other parts of the first contour (400, 500) not changed by the user.

7. The method of any one of claims 1 - 6,
wherein extracting (140) comprises extracting (140) the positive samples (322) as one or more patches in the medical image (350) including the part (430) of the first contour (400, 500) after the change and extracting (140) the negative samples (324) as one or more patches in the medical image (350) spaced from the first contour (400, 500) after the change; and/or
wherein extracting (140) comprises extracting (140) the negative samples (324) as one or more patches in the medical image (350) including the part of the first contour (420, 500) before the change.

8. The method of any one of claims 1 - 7,
wherein segmenting (150) resulting in the second contour (400, 520) comprises segmenting (150) by the first machine-learned model (360) comprising a U-Net.

9. The method of any one of claims 1 - 8,
wherein segmenting (150) resulting in the second contour (400, 520) comprises:
forming (152) a contrastive weight map (340) from the positive and negative samples (322, 324) and
outputting the second contour (400, 520) by the first machine-learned model (360) in response to input of the medical image (350) and the information, the information comprising the contrastive weight map (340).

10. The method of claim 9,
wherein forming (152) comprises, in particular, for each of different location samples from the medical image (350), forming (152) a weight from a maximization of similarity of the location sample with the positive samples (322) as inversely weighted by similarity of the location sample with the negative samples (324), and/or
wherein forming (152) comprises, in particular, forming (152) the contrastive weight map (340) from embedded features derived from the positive and negative samples (322, 324) by an encoder.

11. The method of claim 9 or 10, wherein the contrastive weight map (340) is based on similarity of locations in the medical image (350) with the positive samples (322) and dissimilarity with the negative samples (324).

12. The method of any one of claims 1 - 11, wherein the information comprises a relational map (340) formed with the positive and negative samples (322, 324).

13. A medical system for interactive segmentation, the medical system comprising:
a memory (610) configured to store a medical image (350);
a user input (620) configured to receive an alteration from a user;
an image processor (600) configured to receive an alteration from a first location (410A) to a second location (410B) for a first segmentation of the medical image (350) from the user input, and carry out the method of any one of claims 1 - 12 to generate a second segmentation (370) of the medical image (350), the second segmentation (370) formed by a machine-learned network (360); and
a display (630) configured to display a segmentation image of the second segmentation (370).

14. The medical system of claim 13, wherein the alteration is a change in position of a border (400) of the first segmentation where the negative samples (324) are at locations of the border prior to the change and the positive samples (322) are at locations of the border after the change.

15. The medical system of claim 13 or 14, wherein the machine-learned network (360) comprises an image-to-image network with a first input for the medical image (350) and a second input for a relational map (340) relating positions in the medical image (350) with similarity with the positive samples and the negative samples (322, 324).

16. The medical system of any one of claims 13 - 15,
wherein the image processor (600) is configured to generate feature embeddings (332, 334, 336) for the positive samples (322), the negative samples (324), and patches (326) from different positions in the medical image (350), the relational map (340) formed of weights maximizing similarity of the feature embeddings of the patches (326) at the different positions with the feature embeddings of the positive samples (322) weighted by similarity of the feature embeddings of the patches (326) at the different positions with the feature embeddings of the negative samples (324).

## Patentansprüche

1. Verfahren zur Segmentierungsunterstützung in einem medizinischen Bildgebungssystem, wobei das Verfahren Folgendes umfasst:
Erfassen (100) eines medizinischen Bilds (350) eines Patienten;
Segmentieren (120) eines Objekts in dem medizinischen Bild (350), wobei das Segmentieren zu einer ersten Kontur (420, 500) für das Objekt führt;
Empfangen (130), aus einer Benutzereingabe, einer Änderung an einem Teil (430) der ersten Kontur (420, 500) für das Objekt;
Extrahieren (140) positiver und negativer Proben (322, 324) aus dem medizinischen Bild (350) basierend auf der geänderten Kontur (400, 510);
Segmentieren (150) des Objekts in dem medizinischen Bild (350), wobei das Segmentieren zu einer zweiten Kontur (520) durch eine Eingabe des medizinischen Bilds (350) und von Informationen für die positiven und negativen Proben (322, 324) in ein erstes maschinell gelerntes Modell (360) führt; und
Erzeugen (160) eines Bilds der zweiten Kontur (520).

2. Verfahren nach Anspruch 1, wobei das medizinische Bild (350) ein Magnetresonanzbild, ein Ultraschallbild, ein Computertomographiebild oder ein Röntgenbild umfasst.

3. Verfahren nach Anspruch 2, wobei das Erfassen (100) eines medizinischen Bilds (350) das Erfassen (100) eines Bilds der intrakardialen Echokardiographie, ICE-Bilds, mit einem ICE-Wandler umfasst, wobei das ICE-Bild (350) ein Herzgebiet des Patienten repräsentiert, wobei das Objekt ein Ostium, eine Herzkammer oder eine Klappe umfasst.

4. Verfahren nach einem der Ansprüche 1-3, wobei das Segmentieren (120), das zu der ersten Kontur (500) führt, das Segmentieren (120) durch ein zweites maschinell gelerntes Modell (605) umfasst, das die erste Kontur (500) als Reaktion auf eine Eingabe des medizinischen Bilds (350) ausgibt.

5. Verfahren nach einem der Ansprüche 1-4, wobei das Empfangen (130) das Empfangen (130) einer Benutzereingabeänderung umfasst, durch die ein Ort (410A) eines Punkts auf der ersten Kontur (420, 500) von der ersten Kontur (420, 500) weg verschoben wird, wobei der Teil (430) den Ort (410A) und benachbarte Orte, die mit der Änderung verschoben werden, umfasst, wobei andere Teile der ersten Kontur (400, 500) frei von der Änderung sind.

6. Verfahren nach Anspruch 5, wobei das Segmentieren (150) das Segmentieren der gesamten ersten Kontur (420, 500), das Bereitstellen der zweiten Kontur (400, 520) für den von dem Benutzer geänderten Teil (430) und andere von dem Benutzer nicht geänderte Teile der ersten Kontur (400, 500) umfasst.

7. Verfahren nach einem der Ansprüche 1-6,
wobei das Extrahieren (140) das Extrahieren (140) der positiven Proben (322) als ein oder mehrere Patches in dem medizinischen Bild (350) mit dem Teil (430) der ersten Kontur (400, 500) nach der Änderung und das Extrahieren (140) der negativen Proben (324) als ein oder mehrere Patches in dem medizinischen Bild (350) mit Abstand von der ersten Kontur (400, 500) nach der Änderung umfasst; und/oder
wobei das Extrahieren (140) das Extrahieren (140) der negativen Proben (324) als ein oder mehrere Patches in dem medizinischen Bild (350) mit dem Teil der ersten Kontur (420, 500) vor der Änderung umfasst.

8. Verfahren nach einem der Ansprüche 1-7,
wobei das Segmentieren (150), das zu der zweiten Kontur (400, 520) führt, das Segmentieren (150) durch das erste maschinell gelernte Modell (360), das ein U-Netz umfasst, umfasst.

9. Verfahren nach einem der Ansprüche 1-8,
wobei das Segmentieren (150), das zu der zweiten Kontur (400, 520) führt, Folgendes umfasst:
Bilden (152) einer Karte (340) kontrastiver Gewichte aus den positiven und negativen Proben (322, 324) und
Ausgeben der zweiten Kontur (400, 520) durch das erste maschinell gelernte Modell (360) als Reaktion auf eine Eingabe des medizinischen Bilds (350) und der Informationen, wobei die Informationen die Karte (340) kontrastiver Gewichte umfassen.

10. Verfahren nach Anspruch 9,
wobei das Bilden (152) insbesondere für jede von verschiedenen Ortsproben aus dem medizinischen Bild (350) das Bilden (152) eines Gewichts aus einer Maximierung einer Ähnlichkeit der Ortsprobe mit den positiven Proben (322) wie umgekehrt gewichtet durch eine Ähnlichkeit der Ortsprobe mit den negativen Proben (324) umfasst und/oder
wobei das Bilden (152) insbesondere das Bilden (152) der Karte (340) kontrastiver Gewichte aus eingebetteten Merkmalen umfasst, die von den positiven und negativen Proben (322, 324) durch einen Codierer abgeleitet werden.

11. Verfahren nach Anspruch 9 oder 10, wobei die Karte (340) kontrastiver Gewichte auf einer Ähnlichkeit von Orten in dem medizinischen Bild (350) mit den positiven Proben (322) und einer Unähnlichkeit mit den negativen Proben (324) basiert.

12. Verfahren nach einem der Ansprüche 1-11, wobei die Informationen eine relationale Karte (340) umfassen, die mit den positiven und negativen Proben (322, 324) gebildet ist.

13. Medizinisches System zur interaktiven Segmentierung, wobei das medizinische System Folgendes umfasst:
einen Speicher (610), der dazu ausgelegt ist, ein medizinisches Bild (350) zu speichern;
eine Benutzereingabe (620), die dazu ausgelegt ist, eine Veränderung von einem Benutzer zu empfangen;
einen Bildprozessor (600), der dazu ausgelegt ist, eine Veränderung von einem ersten Ort (410A) zu einem zweiten Ort (410B) für eine erste Segmentierung des medizinischen Bilds (350) aus der Benutzereingabe zu empfangen und das Verfahren nach einem der Ansprüche 1-12 auszuführen, um eine zweite Segmentierung (370) des medizinischen Bilds (350) zu erzeugen, wobei die zweite Segmentierung (370) durch ein maschinell gelerntes Netz (360) gebildet wird; und
eine Anzeige (630), die dazu ausgelegt ist, ein Segmentierungsbild der zweiten Segmentierung (370) anzuzeigen.

14. Medizinisches System nach Anspruch 13, wobei die Veränderung eine Änderung einer Position einer Grenze (400) der ersten Segmentierung ist, wobei die negativen Proben (324) an Orten der Grenze vor der Änderung sind und die positiven Proben (322) an Orten der Grenze nach der Änderung sind.

15. Medizinisches System nach Anspruch 13 oder 14, wobei das maschinell gelernte Netz (360) ein Bild-zu-Bild-Netz mit einer ersten Eingabe für das medizinische Bild (350) und einer zweiten Eingabe für eine relationale Karte (340) umfasst, die Positionen in dem medizinischen Bild (350) mit einer Ähnlichkeit mit den positiven Proben und den negativen Proben (322, 324) in Beziehung setzt.

16. Medizinisches System nach einem der Ansprüche 13-15, wobei der Bildprozessor (600) dazu ausgelegt ist, Merkmalseinbettungen (332, 334, 336) für die positiven Proben (322), die negativen Proben (324) und Patches (326) von verschiedenen Positionen in dem medizinischen Bild (350) zu erzeugen, wobei die relationale Karte (340) aus Gewichten gebildet ist, die eine Ähnlichkeit der Merkmaleinbettungen der Patches (326) an den verschiedenen Positionen mit den Merkmaleinbettungen der positiven Proben (322) gewichtet durch eine Ähnlichkeit der Merkmaleinbettungen der Patches (326) an den verschiedenen Positionen mit den Merkmaleinbettungen der negativen Proben (324) maximieren.

## Revendications

1. Procédé d'aide à la segmentation dans un système d'imagerie médicale, le procédé comprenant :
l'acquisition (100) d'une image médicale (350) d'un patient ;
la segmentation (120) d'un objet dans l'image médicale (350), la segmentation conduisant à un premier contour (420, 500) pour l'objet ;
la réception (130), à partir d'une entrée utilisateur, d'un changement dans une partie (430) du premier contour (420, 500) pour l'objet ;
l'extraction (140) des échantillons positifs et négatifs (322, 324) de l'image médicale (350) sur la base du contour modifié (400, 510) ;
la segmentation (150) de l'objet dans l'image médicale (350), la segmentation conduisant à un second contour (520) par entrée de l'image médicale (350) et des informations pour les échantillons positifs et négatifs (322, 324) dans un premier modèle appris par machine (360) ; et
la génération (160) d'une image du second contour (520).

2. Procédé selon la revendication 1, dans lequel l'image médicale (350) comprend une image à résonance magnétique, une image ultrasonore, une image tomographique assistée par ordinateur ou une image radiographique.

3. Procédé selon la revendication 2, dans lequel l'acquisition (100) d'une image médicale (350) comprend l'acquisition (100) d'une image d'échocardiographie intracardiaque, ICE, avec un transducteur ICE, l'image ICE (350) représentant une région cardiaque du patient, dans lequel l'objet comprend un ostium, une chambre cardiaque ou une valve.

4. Procédé selon l'une quelconque des revendications 1 à 3, dans lequel la segmentation (120) conduisant au premier contour (500) comprend la segmentation (120) par un second modèle appris par machine (605) délivrant le premier contour (500) en réponse à l'entrée de l'image médicale (350).

5. Procédé selon l'une quelconque des revendications 1 à 4, dans lequel la réception (130) comprend la réception (130) d'un changement d'entrée utilisateur décalant un emplacement (410A) d'un point sur le premier contour (420, 500) à l'écart du premier contour (420, 500), la partie (430) comprenant l'emplacement (410A) et des emplacements adjacents décalés avec le changement où d'autres parties du premier contour (400, 500) sont exemptes de changement.

6. Procédé selon la revendication 5, dans lequel la segmentation (150) comprend la segmentation de l'ensemble du premier contour (420, 500), la fourniture du second contour (400, 520) pour la partie (430) modifiée par l'utilisateur et d'autres parties du premier contour (400, 500) non modifiées par l'utilisateur.

7. Procédé selon l'une quelconque des revendications 1 à 6,
dans lequel l'extraction (140) comprend l'extraction (140) des échantillons positifs (322) sous la forme d'un ou plusieurs correctifs dans l'image médicale (350) comprenant la partie (430) du premier contour (400, 500) après le changement et l'extraction (140) des échantillons négatifs (324) sous la forme d'un ou plusieurs correctifs dans l'image médicale (350) espacés du premier contour (400, 500) après le changement ; et/ou
dans lequel l'extraction (140) comprend l'extraction (140) des échantillons négatifs (324) sous la forme d'un ou plusieurs correctifs dans l'image médicale (350) incluant la partie du premier contour (420, 500) avant le changement.

8. Procédé selon l'une quelconque des revendications 1 à 7,
dans lequel la segmentation (150) conduisant au second contour (400, 520) comprend la segmentation (150) par le premier modèle appris par machine (360) comprenant un U-Net.

9. Procédé selon l'une quelconque des revendications 1 à 8,
dans lequel la segmentation (150) conduisant au second contour (400, 520) comprend :
la formation (152) d'une carte de poids contrastive (340) à partir des échantillons positifs et négatifs (322, 324) et
la délivrance du second contour (400, 520) par le premier modèle appris par machine (360) en réponse à l'entrée de l'image médicale (350) et des informations, les informations comprenant la carte de poids contrastive (340).

10. Procédé selon la revendication 9,
dans lequel la formation (152) comprend, en particulier, pour chacun des différents échantillons d'emplacement provenant de l'image médicale (350), la formation (152) d'un poids à partir d'une maximisation de similarité de l'échantillon d'emplacement avec les échantillons positifs (322) comme pondéré inversement par similarité de l'échantillon d'emplacement avec les échantillons négatifs (324), et/ou
dans lequel le terme (152) comprend, en particulier, la formation (152) de la carte de pondération contrastive (340) à partir de caractéristiques incorporées dérivées des échantillons positifs et négatifs (322, 324) par un codeur.

11. Procédé selon la revendication 9 ou 10, dans lequel la carte de poids contrastive (340) est basée sur la similarité des emplacements dans l'image médicale (350) avec les échantillons positifs (322) et la dissimilarité avec les échantillons négatifs (324).

12. Procédé selon l'une quelconque des revendications 1 à 11, dans lequel les informations comprennent une carte relationnelle (340) formée avec les échantillons positifs et négatifs (322, 324).

13. Système médical de segmentation interactive, le système médical comprenant :
une mémoire (610) configurée pour stocker une image médicale (350) ;
une entrée utilisateur (620) configurée pour recevoir une modification d'un utilisateur ;
un processeur d'image (600) configuré pour recevoir une modification d'un premier emplacement (410A) à un second emplacement (410B) pour une première segmentation de l'image médicale (350) à partir de l'entrée utilisateur, et exécuter le procédé selon l'une quelconque des revendications 1 à 12 pour générer une seconde segmentation (370) de l'image médicale (350), la seconde segmentation (370) étant formée par un réseau appris par machine (360) ; et
un afficheur (630) configuré pour afficher une image de segmentation de la seconde segmentation (370).

14. Système médical selon la revendication 13, dans lequel la modification est un changement de position d'une bordure (400) de la première segmentation où les échantillons négatifs (324) se trouvent à des emplacements de la bordure avant le changement et les échantillons positifs (322) se trouvent à des emplacements de la bordure après le changement.

15. Système médical selon la revendication 13 ou 14, dans lequel le réseau appris par machine (360) comprend un réseau d'image à image avec une première entrée pour l'image médicale (350) et une seconde entrée pour une carte relationnelle (340) concernant des positions dans l'image médicale (350) avec une similarité avec les échantillons positifs et les échantillons négatifs (322, 324).

16. Système médical selon l'une quelconque des revendications 13 à 15, dans lequel le processeur d'image (600) est configuré pour générer des incorporations de caractéristiques (332, 334, 336) pour les échantillons positifs (322), les échantillons négatifs (324) et des correctifs (326) provenant de différentes positions dans l'image médicale (350), la carte relationnelle (340) étant formée de poids maximisant la similarité des incorporations de caractéristiques des correctifs (326) aux différentes positions avec les incorporations de caractéristiques des échantillons positifs (322) pondérées par similarité des incorporations de caractéristiques des correctifs (326) aux différentes positions avec les incorporations de caractéristiques des échantillons négatifs (324).
